(19) Europäisches Patentamt
European Patent Office
Office européen des brevets

(11) Publication number: **0 192 976**
**B1**

(12) # EUROPEAN PATENT SPECIFICATION

(45) Date of publication of patent specification: **14.06.89**

(21) Application number: **86101064.3**

(22) Date of filing: **27.01.86**

(51) Int. Cl.$^4$: **C 07 D 213/82,** C 07 D 241/24, C 07 D 213/89, C 07 D 231/14, C 07 D 261/18, A 61 K 31/455, A 61 K 31/495, A 61 K 31/415, A 61 K 31/42

(54) **Compounds having antihyperlipemic activity, processes for the preparation thereof and pharmaceutical compositions containing them.**

(30) Priority: **28.01.85 IT 1925185**

(43) Date of publication of application: **03.09.86 Bulletin 86/36**

(45) Publication of the grant of the patent: **14.06.89 Bulletin 89/24**

(84) Designated Contracting States: **AT BE CH DE FR GB IT LI LU NL SE**

(56) References cited:
**EP-A-0 053 681**
**EP-A-0 064 286**
**FR-A-2 146 138**
**US-A-4 021 436**
**US-A-4 278 680**

(73) Proprietor: **BOEHRINGER BIOCHEMIA ROBIN S.p.A.**
**Via S. Uguzzone, 5**
**I-20126 Milan (IT)**

(72) Inventor: **Tofanetti, Odoardo**
**Via S. Uguzzone, 5**
**I-20126 Milan (IT)**
Inventor: **Spinelli, Silvano**
**Via S. Uguzzone, 5**
**I-20126 Milan (IT)**
Inventor: **Casciarri, Igino**
**Via S. Uguzzone, 5**
**I-20126 Milan (IT)**

(74) Representative: **Bianchetti, Giuseppe**
**Studio Consulenza Brevettuale Via Rossini, 8**
**I-20122 Milan (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 192 976 B1

## Description

The present invention relates to therapeutically useful compounds as antihyperlipemic agents, a process for the preparation thereof and pharmaceutical compositions containing them as active ingredients.

Particularly, the present invention relates to 1,10-bis[2-(hydroxyethyl)thio]decane esters with N-heteroaryl-aminoacids (e.g. nicotinuric acid or nicotinoylglycine and analogues) having the following formula I:

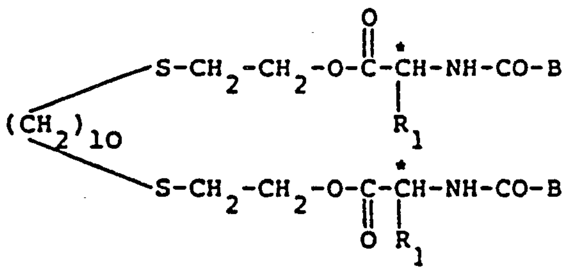

(I)

wherein $R_1$ is hydrogen, methyl, ethyl, isopropyl, isobutyl, 2-butyl, hydroxymethyl, 1-hydroxy-ethyl, carboxymethyl, aminocarbonylmethyl, 2-carboxy-ethyl, 2-aminocarbonylethyl, 4-amino-butyl, 4-amino-3-hydroxy-butyl, 5-imidazolyl-methyl, 3-guanidino-propyl, phenyl, benzyl, 4-hydroxy-benzyl, 3-indolyl-methyl, (4-hydroxy-3,5-diiodophenoxy)-3,5-diiodobenzyl, thiomethyl, methylthioethyl or a group of formula

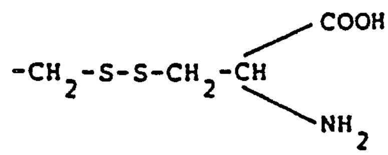

;

when $R_1$ is different from hydrogen, the carbon atoms marked with an asterisk may be in L, D or DL form;

B is an heteroaryl residue selected from the group comprising 3-pyridyl, 5-bromo-3-pyridyl, 5-fluoro-3-pyridyl, pyrazinyl, 5-methyl-pyrazinyl and N-oxides thereof, 5-methyl-pyrazol-3-yl and 5-methyl-isoxazol-3-yl.

The present invention also relates to pharmaceutically and veterinarily acceptable inorganic and organic acid addition salts of compounds of formula I.

Examples of therapeutically acceptable salts are the salts with inorganic acids, such as hydrochloric, sulphuric and phosphoric acids, and organic acids, such as methanesulphonic, malic, fumaric and tartaric acids.

Compounds I may be used in therapy, in the treatment of atherosclerosis, a multifactorial disease characterized by a decrease in arterial elasticity, thickening and stiffness of arterias and by lesions of great or median arteries with deposit of atheromatous plaques containing cholesterol and lipid materials (phospholipids, lipoproteins and cholesterol).

Therefore, in patients affected with primary hyperlipemia, i.e. hyperlipemia not resulting from other disturbances, such as lipid nephrosis, diabetes or hypothyroidism, the treatment consists in reducing plasma concentrations of cholesterol, lipoproteins (particularly LDL and VLDL) and triglycerides, by means of dietary measures, to be advised according to the single case, or, with the progress and aggravation of the disease, in a treatment with hypolipemizing drugs commonly used in therapy.

On the other hand, at least five types of hyperlipemia are known, according to Friedrickson's classification, but no drug equally effective on all of the five types of hyperlipemia is hitherto known.

Thus, for instance, clofibric acid and its similar compounds are effective against type-III, IV and V hyperlipemias, whilst bezafibrate, a compound of the same group of clofibric acid, seems to be useful also in type-II hyperlipemia.

Bis-hydroxyethylthio-1,10-decane (thiadenol), which is able to block endogenous synthesis of cholesterol at both hepatic and intestinal level, is particularly effective in type-II and IV hyperlipemia, but it shows a very low half-life time, which involves frequent administrations of the drug.

Another thioether, probucol, is particularly useful in the treatment of type-II hyperlipemia and all the disturbances involving high cholesterol concentrations.

Compounds, such as pantetine disulfide or 3-hydroxy-3-methylglutaric acid, are useful in the treatament of type-IIa and -IIb dyslipemias, by interference in the biochemical processes leading to cholesterol synthesis. Other drugs such as cholestyramine and glucurono-2-amino-2-deoxyglycane sulfate are used in therapy due to their ability to inhibit respectively cholesterol absorption at the intestinal level and VLDL lipoproteins uptake by the arterial walls, therefore contributing in lowering blood viscosity and platelets adhesivity.

Finally, a particularly therapeutically useful group of drugs is that of nicotinic acid and nitocinic-like activity compounds, such as nicotinic acid N-oxide, 5-bromo- and 5-fluoro-nicotinic acids, alkyl-substituted nicotinic acids; pyrazinoic acid, 5-methyl-pyrazinoic acid and N-oxides thereof; 5-methyl-pyrazol- and 5-methylisoxazol-3-carboxylic acids and corresponding amides, alcohols, ethers and/or thioethers (wherein the COOH group is replaced by $CONH_2$, $CH_2OH$, $CH_2OAlkyl$, $CH_2S$ Alkyl).

These compounds have a different mechanism of action from that of the above mentioned compounds, which mainly consists in an antilipolytic effect, reducing the concentrations of phospholipids (VLDL, LDL), free fatty acids, triglycerides and cholesterol in the circulation.

Nicotinic acid and similar compounds, in fact, increase cholesterol bile secretion but not that of bile acids. In therapy, nicotinic acid must be administered in high dosages (3 to 6 g a day), which frequently causes marked side-effects, such as gastric irritation, pruritus, intense cutaneous fluch on face and limbs, and cephalgia.

Since nicotinic acid and similar compounds are rapidly excreted, they must be adminstered in high dosages, to obtain effective concentrations of the drug in the blood, warranting on therapeutic efficacy.

Alternatively, nicotinic acid and/or similar compounds may be administered in form of amides, ethers or bonded with polymers releasing slowly the active ingredient, in order to decrease the absorption and excretion rates of the drug, thus improving the therapeutic index of said compounds and prolonging the plasmatic half-life, at the same time reducing the above described untoward effects.

That seems to be particularly important, because the nicotinic acid, at therapeutically effective blood levels, was shown to exert a profibrinolytic activity.

With this respect, US Patent 4,021,436, FR—A—2,146,138, FR—A—2,324,303, EP—A—0053681 and EP—A—0060994 describe nicotinic esters or nicotinoyl carbonates of bis-hydroxyethylthio-1,10-decane, in order to combine the proved effectiveness of bis-hydroxyethylthio-1,10-decane in normalizing the impaired lipidic metabolism of atherosclerotic patients, with the peculiar therapeutic characteristics of nicotinic acid, thus attaining a reduction in total lipids (particularly those bonded to LDL and VLDL), triglycerides and cholesterol.

The known esters of nicotinic acid and bis-hydroxyethylthio-1,10-decane should release "in vivo" the single constituents, by the action of plasmatic esterases, thus increasing and enlarging the therapeutic action of each of them.

Particularly for the nicotinic ester of bis-(hydroxyethylthio)-1,10-decane, it is reported that the oral administration of the new molecule, in comparison with equal doses of the single compounds, nicotinic acid and bis-(hydroxyethylthio)-1,10-decane, allows to obtain better therapeutic indexes as for $DL_{50}$ values.

Moreover it is known that nicotinuric acid (nicotinoylglycine), one of the main urinary metabolites of nicotinic acid, is able to lower plasmatic concentrations of cholesterol, when administered orally (T. A. Mietirmen, Atherosclerosis, Proceed. Sec. Int. Symp, R. J. Jones Ed., Springer N.Y., *1970*, 508).

Unfortunately nicotinuric acid, though pharmaceutically effective per se, cannot be used in human therapy, due to its lower adsorption than that of nicotinic acid.

It has been now surprisingly found that compounds I, according to the invention, besides having a high hypolipemizing activity and negligible side-effects, are effectively adsorbed by the oral route, attaining long-lasting therapeutically effective hematic concentrations. Moreover the compounds of the present invention, contrarily to thiadenol and nicotinic acid known derivatives, show no "rebound" effect, which is a paradoxal increase in unesterified fatty acids and glycerol above the initial values, consequent to interruption of the drug treatment.

Among the compounds of formula I, particularly preferred are those in which B is 3-pyridyl, 5-bromo-pyridyl, pyrazinyl or the corresponding N-oxides thereof; $R_1$ is selected from the group consisting of hydrogen, methyl, benzyl, methylthioethyl, isopropyl, isobutyl, 2-butyl, phenyl, i.e. from compounds deriving from the amino acids glycine, alanine, phenylalanine, methionine, valine, leucine, isoleucine and phenylglycine, preferably in the L form.

Particularly preferred compounds of the invention are:

1,10-bis[2-(nicotinoylglycyl)ethylthio]decane;

1,10-bis[2-(5′-bromo-nicotinoylglycyl)ethylthio]decane;

1,10-bis[2-(nicotinoylalanyl)ethylthio]decane.

The compounds according to the present invention may be prepared by reacting 1,10-bis[2-(hydroxyethyl)thio]decane (II), with a compound of general formula (III):

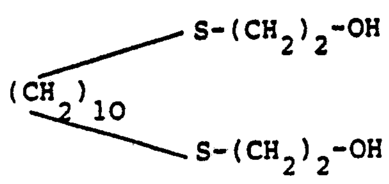

(II)

+

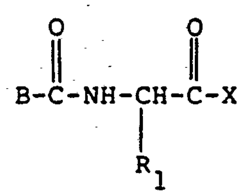

(III)

wherein B and $R_1$ have the above mentioned meanings, and X is OH or N,N-bis(2-oxo-3-oxazolidinyl)-phosphorodiamidyl.

Alternatively, compounds of general formula I may be prepared by reaction of a compound of general formula (IV):

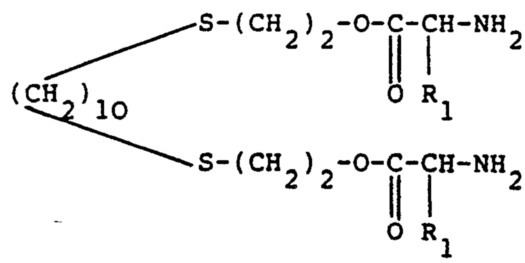

(IV)

wherein $R_1$ has the above mentioned meanings, with a compound of general formula (V):

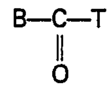

(V)

wherein B has the above mentioned meanings and T is a member selected from the group consisting of: OH, Cl, $N_3$, N,N-bis(2-oxo-3-oxazolidinyl)phosphorodiamidyl

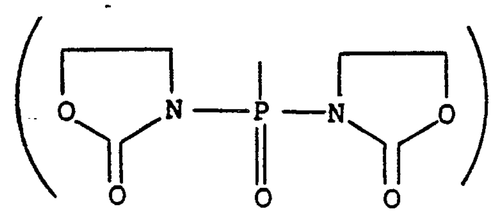
, 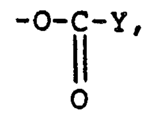
,

Y being a member selected from the group consisting of ($C_1$—$C_5$)alkyl, benzyl, p-nitrophenyl, hydroxysuccinimide, hydroxybenzotriazole.

Compounds of general formula IV may be obtained from compounds of general formula (VI):

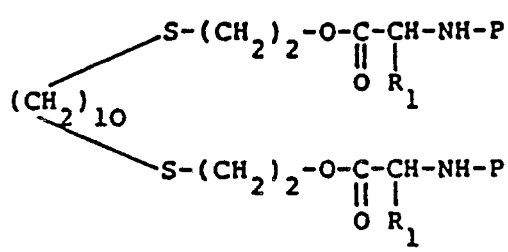

(VI)

wherein $R_1$ has the above mentioned meanings and P is a protecting group for the amino residue, e.g. a formyl or tert-butoxycarbonyl (BOC) group.

Compounds of general formula VI may be prepared on their turn by reaction of 1,10-bis[2-(hydroxyethyl)thio]decane with compounds of general formula VII

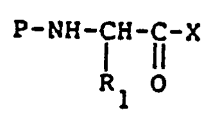

(VII)

wherein $R_1$, P and X have the above mentioned meanings.

Compound of formula II, 1,10-bis[2-(hydroxyethyl)thio]decane, is disclosed in FR—A—2146138 (Chem. Abstr. 79, 41919f).

Compounds of general formula III, wherein X = OH, are prepared from nicotinic, bromonicotinic, fluoronicotinic, pyrazinoic, 5-methyl-pyrazinoic acids and commercially available N-oxides thereof, by reaction with methyl, ethyl or t-butyl esters of the aminoacids according to the invention, substantially following the method described by M. T. Wu and R. E. Lyle in J. Pharm. Sci. *61,* 141—2 (1972); the resulting amides are then hydrolyzed to give the corresponding free acids.

The esterification of a compound of general formula III, wherein X = OH, with compound II, is preferably carried out in the presence of carbodiimides, particularly dicyclohexylcarbodiimide (DCCD), in stoichiometric amounts or in a slight excess, preferably 10% molar excess, and 4-dimethylaminopyridine

(DMAP) in catalytic amounts, preferably 10% molar with respect to carbodiimide. The reaction may be carried out in aprotic organic solvents, such as methylene chloride, dichloroethane, diethylenedioxide, tetrahydrofuran, diglyme, dimethoxyethane, dimethylsulfoxide, dimethylformamide, the latter being particularly preferred.

The reaction may be carried out at a temperature ranging from 0° to 50°C, preferably at room temperature, and it is complete within 3—48 hours; usually within 12 hours.

Alternatively, compounds of formula III wherein X is OH, are transformed into the corresponding derivatives in which X is a group of formula

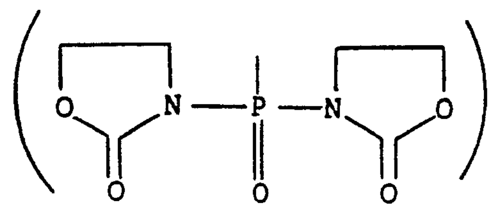

and subsequently reacted with compounds of formula II, according to the procedure of M. Ballester-Rodès and A. L. Palomo et al. in Synth. Comm. 515 (1984), to give compounds of general formula (I).

The reaction between compounds of general formula IV and compounds of general formula V may be carried out using acyl chlorides of compounds V (T = Cl), prepared from the corresponding nicotinic, bromonicotinic, fluoronicotinic, pyrazinoic, 5-methyl-pyrazinoic acids or the N-oxides thereof, according to the method described by R. Wingfield et al. J. Am. Chem. Soc. *75,* 4364 (1953).

Compounds of formula V, wherein T is Cl, are then reacted with compounds of general formula IV, in an inert organic solvent such as dichloroethane, methylene chloride, benzene, or, preferably, toluene, in the presence of a tertiary organic base, such as triethylamine, tributylamine, N,N-dimethylaniline, N,N,N,N-tetramethylethylenediamine, preferably triethylamine, in inert atmosphere. The reaction is preferably carried out at a temperature of 0°C.

Alternatively, compounds of general formula V, wherein T is N,N-bis(2-oxo-3-oxazolidinyl)phosphorodiamidyl, are reacted with compounds of formula IV, according to Ballester-Rodès et al. Synth. Comm. 515 (1984).

The deprotection reaction of the amino group of compounds of general formula VI to obtain compounds of general formula IV, is carried out by solubilizing compounds VI in an appropriate organic solvent, selected from the group consisting of methylcellosolve® (methylglykol); isopropyl, ethyl, methyl alcohols; diglyme, or, preferably, anhydrous methanol, and dropping therein a mineral acid solution, preferably hydrochloric acid in the same solvent, at temperatures ranging from 0° to 25°C, preferably 10—15°C.

The reaction is complete at room temperature, within a period from 12 to 48 hours, preferably the reaction mixture is worked up after 12 hours.

Compounds of general formula VII, wherein X is OH, are known in the literature (Chemistry of amino acids: pag. 922; Schnorremberg and Steglich: Angew. Chem. Int. Ed. Engl. *18,* 307 (1979)).

Compounds of general formula VI are prepared by reacting II with said compounds VII in the presence of carbodiimides, preferably dicyclohexylcarbodiimide, in stoichiometric amounts or in slight excess with respect to VII, preferably in 10% molar excess, and 4-dimethylaminopyridine in a catalytic amount, preferably in a 10% molar amount with respect to carbodiimide.

The reaction may be carried out in aprotic organic solvents such as, for instance, methylene chloride, dichloroethane, diethylenedioxide, tetrahydrofuran, diglyme, dimethoxyethane, dimethylsulfoxide; dimethylformamide being preferred.

The reaction may be carried out at a temperature ranging from 0° to 50°C, preferably at room temperature, and it is complete within 3—48 hours, preferably within 12 hours.

Alternatively, compounds of formula VII, wherein X is OH, are transformed into the corresponding derivatives in which X is a group of formula

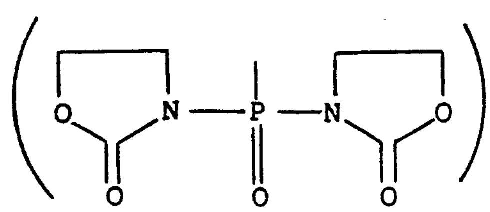

and subsequently reacted with II, according to M. Ballester-Rodès and A. L. Palomo et al. in Synth. Comm. 515 (1984).

Compound (1), 1,10-bis[2-(N-nicotinoylglycyl)ethylthio]decane, was selected as a typical representative example of the compounds of the invention. Pharmacological and toxicological characteristics of compound (1) were evaluated in comparison to nicotinic acid (2); a 1,10-bis[2-(hydroxyethyl)thio]-decane (3) and the esters 1,10-bis[2-nicotinoyl)ethylthio]decane (4) and 1,10-bis[2-(nicotinoyloxymethylcarbonyloxy)ethylthio]decane (5).

Compound 4, described in the above mentioned patents U.S. 4,021,436, FR—A—2,146,138, FR—A—2,324,303, EP—A—0053681 and 0060994, and compound 5, which is structurally similar, may be considered as the closest prior art.

The formulae of said compounds are reported hereinbelow

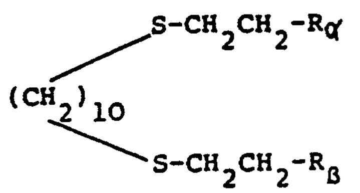

4) $R_\alpha = R_\beta =$ 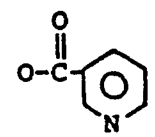

5) $R_\alpha = R_\beta =$ 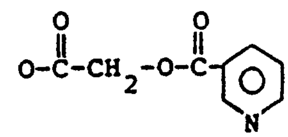

The single compounds were administered orally at single equimolecular gradually increasing doses, in order to comparatively evaluate their hypocholesterolemic and hypotriglyceridemic activities. Normolipidemic, and hypercholesterolemic acid hypertriglyceridemic rats, following to administration of diets respectively enriched in cholesterol and cholic acid (Nath diet) of 79.5% fructose, were used.

Finally, normolipemic rats, housed under normal conditions, were treated for 5 days with the compounds under test, at gradually increasing doses. At the end of said treatment, the rats were fasted for 16 hours and administered with Triton® 1.339, at a dose of 0.5 g/kg i.v. Two hours later, the rats were killed and the parameters in comparison with the animals treated respectively with the sole vehicle and Triton® were evaluated.

Male Sprague-Dawley rats of 180—200 g body weight were used. The compounds under test were administered as 1% tylose suspension, by means of gastric probe, 10 animals per dose in comparison with controls, treated with the sole vehicle.

Four dose levels: 1, 2, 4, $8 \cdot 10^{-3}$ mol/kg were used for each compound under test. The body growth and liver weight were evaluated.

In the serum the following values were determined:

a) total cholesterol, by enzymatic method;

b) HDL cholesterol, by enzymatic method, after precipitation of the other lipoproteins by addition of phosphotungstic acid;

c) triglycerides by enzymatic method, i.e. by means of enzymatic hydrolysis and dosage of the formed glycerol.

The dosage of the liver triglycerides has been carried out by enzymatic method on the evaporated liver extract with chloroform:methanol 2:1.

The results were evaluated and expressed as % variation with respect to the control groups.

In the hypercholesterolemic animals (Nath diet), the comparison compound 1,10-bis[2-(nicotinoyl)ethylthio]decane, in all the assayed doses, induced a marked decrease in the HDL concentration, which decreased with the progressive increasing of the administered dose. Moreover, a 10% preliminary increase of the total cholesterol at a $2 \cdot 10^{-3}$ mol/kg dose, followed by a 50% decrease versus controls, which did not vary increasing the dose, was evidenced.

At all the tested doses, the triglyceride hematic concentration was always higher than that in the control animals, although a tendency to decrease with the increasing of the dose was noted; moreover, a gradual increase in the liver weight was observed.

A similar behaviour was evidenced after administration of 1,10-bis[2-(nicotinoyloxy-acetyl)ethylthio]decane.

As an example, at the dose of $4 \cdot 10^{-3}$ mol/kg, a 30% decrease of total plasmatic cholesterol was noted. Contemporary decrease in hematic HDL cholesterol and triglycerides, respectively of 6 and 34%, combined with an increase in the concentration of hepatic triglycerides.

On the contrary, compound 1,10-bis[2-(nicotinoylglycyl)ethylthio]decane according to the invention, at a dose of $4 \cdot 10^{-3}$ mol/kg, induced a decrease in total cholesterol, hematic and hepatic triglycerides respectively of 37, 4 and 48%, with contemporary 7% increase in HDL cholesterol. At the same time, no substantial changes in the liver weight were observed.

All the induced changes were statistically significant and dose-related, in the considered dose range.

The hypotriglyceridemizing activity of the compounds of the invention, as already mentioned, was evaluated in fructose and Triton® induced hypertriglyceridemias, in comparison with the following standards: 1,10-bis[2-(hydroxyethyl)thio]decane (3), its nicotinate (4) and nicotinoyloxymethylcarbonyloxy ester (5).

All the compounds were administered at equimolecular gradual dosages.

No significant differences among the single compounds in decreasing triglyceride hematic level were evidenced. Said decrease, for instance in the fructose test, was about 50—60% with respect to the control values; for all the compounds at an equimolecular dosage corresponding to 100 mg/kg/day of 1,10-bis[2-(hydroxyethyl)thiodecane]. All the compounds caused a variable but not statistically significant decrease in total cholesterol and HDL cholesterol hematic levels. On the contrary, in the "Triton-test", HDL cholesterol level appeared to be increased, without significant differences among the compounds.

Surprisingly, compound 1,10-bis[2-(nicotinoylglycyl)ethylthio]decane (1), according to the invention, and its O-thioester (5) proved to reduce or keep almost unchanged the triglyceride hepatic concentration, with percent variations from −10 to +10%, with respect to 40—50% increases observed for 1,10-bis[2-(hydroxyethyl)thio]decane (3) and its nicotinate (4).

This surprising and unforeseeable effect on the triglyceride hepatic concentration, is a substantial and favorable feature of the compounds of the invention.

In fact said favorable effect, combined with the contemporary decrease of triglyceride plasmatic concentration, is a sign of a catabolic event on triglycerides, said decrease at plasmatic level not resulting from a redistribution effect of the triglycerides, as it could appear from the increases of the parenchyma.

The antilipolytic activity of the compounds of the invention was studied in comparison with 1,10-bis(2-hydroxyethylthio)decane (3), which showed no activity, its nicotinic ester (4), the ester (5), and nicotinic acid (2). All the standards 2, 4, 5 and the compounds of the invention proved to have antilipolytic activity both under normal feeding conditions and in the lipolysis process induced by fasting or adrenalin.

The compounds were administered orally. The results from some experiments, after administration of compounds in amounts of $1 \cdot 10^{-3}$ mol/kg, in a single dose, are reported in Tables 1, 2, 3. The results are expressed as μEq/l of free fatty acids.

TABLE 1 - Normolipolysis

| Time | Controls | nicotinic acid (2) | 1,10-bis-(hydroxyethylthio)-decane esters | | |
|---|---|---|---|---|---|
| | | | nicotinate (4) | ester (5) | nicotinoyl glycinate (1) |
| Basal | 240 | 260 | 230 | 240 | 230 |
| 2 hours | 170 | 110 | 120 | 120 | 200 |
| 4 hours | 280 | 160 | 155 | 170 | 225 |
| 8 hours | 390 | 560 | 450 | 260 | 270 |

10 Animals per group — Normally fed for 7 days, then administered with the test compounds.

TABLE 2 - Fasting induced lipolysis

| Time | Controls | nicotinic acid (2) | 1,10-bis-(hydroxyethylthio)-decane esters | | |
|---|---|---|---|---|---|
| | | | nicotinate (4) | ester (5) | nicotinoyl glycinate (1) |
| 2 hours | 730 | 180 | 250 | 207 | 480 |
| 4 hours | 750 | 190 | 240 | 228 | 430 |
| 8 hours | 720 | 950 | 970 | 744 | 400 |

10 Animals per group — Normally fed for 6 days, then fasted for 24 hours and orally administered with the test compounds.

TABLE 3 - Adrenalin induced lypolysis

| Compound | FFA µEq/l 30' | 60' |
|---|---|---|
| Control | 310 | 310 |
| Control + adrenalin | 780 | 750 |
| Nicotinic acid (2) | 340 | 650 |
| 1,10-Bis(2-hydroxyethyl-thio)decane nicotinate (4) | 295 | 625 |
| 1,10-Bis(2-hydroxyethyl-thio)decane nicotinoyloxy-methylcarbonyloxy ester (5) | 270 | 610 |
| 1,10-Bis(2-hydroxyethyl-thio)decane nicotinoyl-glycinate (1) | 450 | 400 |

10 Animals per group — Fed and housed for one week, then administered orally and stimulated with adrenalin 1 mg/kg i.p., respectively 30 and 60 min before killing.

It is well known that nicotinic acid finds a limited therapeutic application, due to its peripheral vasodilating action, inducing undesired side-effects such as cephalgia and flushing of the face, which may be so marked and unpleasant as to result in poor patient compliance or even interruption of the treatment.

Such a side-effect may be experimented on male albino Guinea pigs, weighing 300—400 g, normally fed and housed in silent room, by observing the vasodilation of ear marginal veins, after administration of the test compounds.

Table 4 shows the results obtained comparing nicotinic acid and compounds 1,10-bis(2-hydrodyethylthio)decane nicotinic ester (4) and ester (5) at doses of $1 \cdot 10^{-3}$ mol/kg, with 1,10-bis[2-(nicotinoylglycyl)ethylthio]decane (1) ester at doses of 1 and $2 \cdot 10^{-3}$ mol/kg.

TABLE 4

| Vasodilating effect | Nicotinic acid (2) | 1,10-bis(2-hydroxyethyl)thiodecane esters: nicotinate (4) | ester (5) | nicotinoylglycinate (1) 1 and 2 $10^{-3}$ moles |
|---|---|---|---|---|
| Beginning | 3' | 10' | 13' | == |
| Maximal effect | 6' | 25' | 30' | == |
| Beginning of disappearance | 20' | 48' | 51' | == |

The above reported data clearly show the marked antilipolytic activity of the compounds according to

the invention, in comparison with other nicotinic esters of 1,10-bis[(2-hydroxyethyl)thio]decane having similar lipophily.

However, the compounds of the invention exhibit the surprising advantages of a long-lasting antilipolytic effect and the absence of the "rebound" effects, characteristic of the other hypolipidemic drugs. Last, but not least, said compounds show no side-effects (flushing), the therapeutical activity being equal.

The compounds of the present invention, particularly 1,10-bis(2-hydroxyethylthio)decane nicotinoylglycyl, 5-bromo-nicotinoyl-glycyl, 5-methyl-pyrazinoylglycyl, nicotinoylalanyl, nicotonoylisoleucyl and nicotinoylphenylalanyl esters, orally administered at doses from 100 to 250 mg/kg, protect the mouse from thrombosis induced by the administration of arachidonic acid, as well as collagene and adrenalin, according to the procedure by G. di Minno and M. J. Silver, Mouse antithrombotic assay, J. Pharm. Exper. Therap. 225, 57 (1983).

Moreover, the compounds of the invention show a markedly reduced acute toxicity, with very high $DL_{50}$ values, ranging from 3 and 6 g/kg in mice and rats.

The very high antilipolytic activity of the compounds of the invention, as well as the poor toxicity thereof, together, contribute to an evident antithrombotic activity "in vivo" and a clear hypocholesterolemic and hypotriglyceridemic effect. It is particularly significant that the hematic normalization of triglycerides does not induce an increase in their hepatic concentration, thus showing that said effect is not corresponding to a redistribution of the triglycerides from the plasma to the hepatic area. For these reasons, the compounds according to the invention are particularly useful in human therapy for the treatment and prophylaxis of hyperlipemias of different type and origin, as well as various pathological conditions such as atherosclerosis and arteriosclerosis, thrombotic conditions and vasal disturbances.

The compounds of the invention may be formulated in form of pharmaceutical compositions to be administered by oral, intramuscular, subcutaneous and/or sublingual route, at doses from 2.5 to 75 mg/kg/day, depending on the dose, number of administrations, age, weight and general conditions of the patient. The compounds of the invention may be administered orally in form of tablets, capsules, drops or syrups; rectally in form of suppositories; parenterally in form of solutions or suspensions to be administered by subcutaneous, intramuscular or intravenous route.

The pharmaceutical compositions of the compounds of the present invention may be conventionally prepared using usual vehicles and diluents such as water, gelatin, lactose, dextrose, saccharose, mannitol, sorbitol, cellulose, talc, stearic acid, calcium and magnesium stearate, glycols, starch, arabic and tragacanth gum, alginic acid and alginates, lecithin, polysorbate (vegetal oils, etc.).

Said pharmaceutical formulations, which are a further object of the invention, will contain at least one compound of formula I or the corresponding salts, together with pharmaceutically acceptable excipients or diluents, compatible with the other ingredients and non-toxic for the patients.

The active ingredient concentration in monodose formulations such as tablets, may vary from 0.05% to 95%.

The oral formulations may be in form of single dose units, such as capsules, sugar coated pills, lozenges, tablets, containing a pre-established amount of the active ingredient of formula I.

The parenteral formulations comprise aqueous, preferably isotonic, preparations of the active ingredient.

The rectal formulations are in form of monodose suppositories, prepared admixing the active ingredient with one or more conventional excipients, such as cocoa butter.

The following examples further illustrate the invention without limiting it.

## Example 1

33.9 Grams of N-formylglycine and 2.5 g of 4-dimethylaminopyridine were added to a solution of 1,10-bis[2-(hydroxyethyl)thio]decane (47.4 g) in 250 ml of anhydrous dimethylformaide. The resulting solution was cooled to 0°—5°C, under stirring, then, keeping the temperature constant, a solution of dicyclohexylcarbodiimide (68 g) in dimethylformamide (75 ml) was dropped therein. After stirring overnight at room temperature, the resulting suspension was filtered, and the solid on the filter was washed with methylene chloride. The filtrate was concentrated to dryness under vacuum and treated with methylene chloride (250 ml): the organic phase was washed in turn with 10% aqueous HCl (50 ml), water (100 ml), 5% aqueous NaOH (50 ml), finally again with 100 ml of water. After drying over sodium sulfate, and evaporation of the solvent under vacuum, an oil was obtained which, after two crystallizations from ethyl acetate, yielded 60 g of 1,10-bis[2-(N-formylglycyl)ethylthio]decane, m.p. 82—85°C (TLC: $SiO_2$, eluent acetone/methylene chloride 1:1, Rf: 0.5).

## Example 2

The procedure of Example 1 was repeated, using respectively:

N-formyl-alanine
N-formyl-leucine
N-formyl-isoleucine
N-formyl-phenylalanine
N-formyl-methionine

or, alternatively, the t-butoxy-carbonates (BOC) thereof, namely BOC-alanine, BOC-leucine, BOC-isoleucine, BOC-phenylalanine, BOC-methionine. The following compounds were obtained:

1,10-bis[2-(N-formyl-alanyl)-ethylthio]decane, m.p. 86—88°;
1,10-bis[2-(N-formyl-leucyl)-ethylthio]decane, m.p. 90—92°;
1,10-bis[2-(N-formyl-isoleucyl)-ethylthio]decane, m.p. 85—88°;
1,10-bis[2-(N-formyl-phenylalanyl)-ethylthio]decane, m.p. 102—110°;
1,10-bis[2-(N-formyl-methionyl)-ethylthio]decane, m.p. 90—92°;
1,10-bis[2-(N-t-butoxycarbonyl-alanyl)-ethylthio]decane, m.p. 100—102°;
1,10-bis[2-(N-t-butoxycarbonyl-leucyl)ethylthio]decane, m.p. 102—106°;
1,10-bis[2-(N-t-butoxycarbonyl-isoleucyl)-ethylthio]decane, m.p. 100—102·;
1,10-bis[2-(N-t-butoxycarbonyl-phenylalanyl)-ethylthio]decane, m.p. 110—115°;
1,10-bis[2-(N-t-butoxycarbonyl-methionyl)-ethylthio]decane, m.p. 102—104°.

Example 3

A saturated solution of methanolic hydrochloric acid (20 ml) was slowly dropped into a solution of 1,10-bis[2-(N-formyl-glycyl)-ethylthio]decane (10 g), in anhydrous methanol (400 ml), kept at 15°—20°C. After stirring overnight, the solvent was removed under vacuum at a temperature of 30—40° and the solid residue was taken up in methylene chloride (100 ml) and methanol (10 ml). The resulting suspension was left to solidify for some hours, then filtered under nitrogen. Upon recrystallization from methanol/methylene chloride, 1,10-bis[2-(glycyl)ethylthio]decane dihydrochloride (6.8 g), m.p. 180—182° was obtained.

Example 4

The procedure of Example 3 was repeated, using the following compounds:

1,10-bis[2-(N-formyl-alanyl)ethylthio]decane
1,10-bis[2-(N-formyl-leucyl)ethylthio]decane
1,10-bis[2-(N-formyl-phenylalanyl)ethylthio]decane
1,10-bis[2-(N-formyl-isoleucyl)ethylthio]decane
1,10-bis[2-(N-formyl-methionyl)ethylthio]decane

or, alternatively, the analogous tert-butoxycarbonyl derivatives thereof, to obtain, respectively:

1,10-bis[2-(alanyl)ethylthio]decane dihydrochloride, m.p. 190—195°;
1,10-bis[2-(leucyl)ethylthio]decane dihydrochloride, m.p. 185—190°;
1,10-bis[2-(phenylalanyl)ethylthio]decane dihydrochloride, m.p. 200—205°;
1,10-bis[2-(isoleucyl)ethylthio]decane dihydrochloride, m.p. 185—187°;
1,10-bis[2-(methionyl)ethylthio]decane dihydrochloride, m.p. 200—205°.

Example 5

1,10-Bis[2-(glycyl)ethylthio]decane dihydrochloride (4.78 g) was added to a cooled solution of triethylamine (8.3 ml) in anhydrous toluene (250 ml), under strong stirring and nitrogen stream. After cooling to 0—5°C, a solution of 5-bromonicotinoyl chloride (9.24 g) in toluene (200 ml) was dropped into the suspension, keeping the same temperature.

After 2 hours at room temperature, the solvent was removed under vacuum and the residue was partitioned between water and methylene chloride. The organic phase was washed with 5% aqueous sodium bicarbonate, with water again, dried over anhydrous sodium sulfate, then concentrated to dryness.

The resulting residue was crystallized from ethyl acetate. 1,10-Bis[2-(5-bromo-nicotinoyl-glycyl)ethylthio]decane, 4.3 g, m.p. 102—104° was obtained, unitary spot in thin layer chromatography [Rf. 0.3, $SiO_2CH_2Cl_2$/acetone 1:1].

Example 6

The procedure of Example 5 was repeated, using the following compounds:

1,10-bis[2-(alanyl)ethylthio]decane
1,10-bis[2-(phenylalanyl)ethylthio]decane
1,10-bis[2-(leucyl)ethylthio]decane
1,10-bis[2-(isoleucyl)ethylthio]decane
1,10-bis[2-(methionyl)ethylthio]decane.

The following compounds were respectively obtained:

1,10-bis[2-(5-bromo-nicotinoylalanyl)ethylthio]decane, m.p. 95—98°;
1,10-bis[2-(5-bromo-nicotinoylphenylalanyl)ethylthio]decane, m.p. 105—106°;
1,10-bis[2-(5-bromo-nicotinoylleucyl)ethylthio]decane, m.p. 102—104°;
1,10-bis[2-(5-bromo-nicotinoylisoleucyl)ethylthio]decane, m.p. 100—102°;
1,10-bis[2-(5-bromo-nicotinoylmethionyl)ethylthio]decane, m.p. 110—115°.

Example 7

1,10-Bis-[2-(2-alanyl-ethyl)thio]decane dihydrochloride (25 g) was added to a solution of nicotinic acid (12.3 g) in 150 ml of dimethylformamide containing triethylamine (41.5 ml), under stirring and inert

atmosphere. N,N-Bis-(2-oxo-3-oxazolidinyl)phosphorodiamidyl chloride (22.9 g) was subsequently added to the resulting solution, and the reaction mixture was stirred for 12 hours. After concentration to a small volume, the residue was partitioned between a sodium bicarbonate aqueous solution (200 ml) and methylene chloride (250 ml). The organic phase was washed with water again and dried under vacuum: after evaporation of the solvent under vacuum, an oily residue was obtained which was crystallized from ethyl acetate. The resulting 1,10-bis[2-(nicotinoylalanylethyl)thio]decane (45.11 g), m.p. 88—90°C, showed a unitary spot in TLC [Rf. 0.4, $SiO_2$, $CH_2Cl_2$/acetone 1:1].

Example 8

The procedure of Example 7 was repeated, using respectively:
1,10-bis[2-(phenylalanyl)ethylthio]decane
1,10-bis[2-(leucyl)ethylthio]decane
1,10-bis[2-(isoleucyl)ethylthio]decane
1,10-bis[2-(methionyl)ethylthio]decane
to obtain the following compounds:
1,10-bis[2-(nicotinoylphenylalanyl)ethylthio]decane, m.p. 95—98°;
1,10-bis[2-(nicotinoyl-leucyl)ethylthio]decane, m.p. 93—95°;
1,10-bis[2-(nicotinoylisoleucyl)ethylthio]decane, m.p. 93—94°;
1,10-bis[2-(nicotinoylmethionyl)ethylthio]decane, m.p. 94—96°.

Example 9

A solution of dicyclohexylcarbodiimide (4.53 g) in dimethylformamide (10 ml) was added to a solution of 1,10-bis[2-(hydroxyethyl)thio]decane (2.94 g) and nicotinoyl glycine (3.96 g) in dimethylformamide (30 ml), cooled at 0—5°C. The resulting suspension was stirred overnight at room temperature. After the usual working up, 1,10-bis[2-(nicotinoyl-glycyl)ethylthio]decane was obtained, in form of an oil which was crystallized from ethyl acetate, to obtain a white solid (4.32 g), m.p. 88—90°, which was pure in TLC [Rf. 0.4, $SiO_2$, $CH_2Cl_2$/acetone 1:1].

Example 10

Triethylamine (6.06 g) was added to a suspension of 1,10-bis[2-(glycyl)ethylthio]decane dihydrochloride (4.78 g) in anhydrous toluene (250 ml), under strong stirring, in inert atmosphere. After 30 min, a solution of toluene (200 ml) containing nicotinoyl chloride (4.23 g) was dropped therein, keeping a temperature between 10 and 20°. Stirring was continued for 1 hour at room temperature, then the solvent was removed under vacuum and the residue was partitioned between chloroform (200 ml) and water (100 ml). The organic layer was subsequently washed twice with 5% aqueous sodium bicarbonate (50 ml) and with water (50 ml) again.

The solvent was dried over sodium sulfate and removed under vacuum. The residue was crystallized from ethyl acetate, to obtain 4 g of 1,10-bis[2-(N-nicotinoylglycyl)ethylthio]decane, m.p. 88—90°.

Example 11

The procedures of Examples 5 and 10 were repeated, using 1,10-bis[2-(glycyl)ethylthio]decane dihydrochloride and the following acids and/or acyl chlorides: pyrazinoic, 5-methyl-pyrazinoic, nicotinic N-oxide, pyrazinoic N-oxide, 5-methyl-pyrazinoic N-oxide, 5-methyl-pyrazol-3-carboxylic, 5-methyl-isoxazol-3-carboxylic, 5-fluoro-nicotinic, to obtain the following compounds:
1,10-bis[2-(pyrazinoylglycyl)ethylthio]decane
1,10-bis[2-(5-methylpyrazinoylglycyl)ethylthio]decane
1,10-bis[2-(N-oxy-nicotinoylglycyl)ethylthio]decane
1,10-bis[2-(N-oxy-pyrazinoylglycyl)ethylthio]decane
1,10-bis[2-(5-methylpyrazol-3-carbonylglycyl)ethylthio]decane
1,10-bis[2-(5-methyl-isoxazol-3-carbonylglycyl)ethylthio]decane
1,10-bis[2-(5-fluoro-nicotinoylglycyl)ethylthio]decane.

**Claims for the Contracting States: BE CH DE FR GB IT LI LU NL SE**

1. Compounds of formula (I)

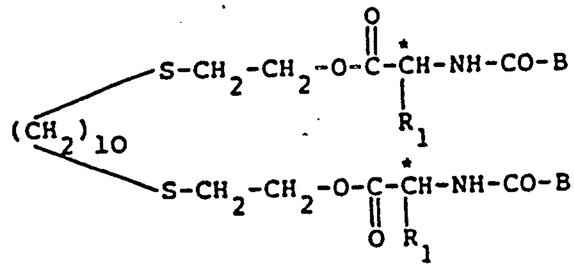

(I)

wherein $R_1$ is hydrogen, methyl, ethyl, isopropyl, isobutyl, 2-butyl, hydroxymethyl, 1-hydroxy-ethyl, carboxymethyl, aminocarbonylmethyl, 2-carboxy-ethyl, 2-aminocarbonylethyl, 4-amino-butyl, 4-amino-3-hydroxy-butyl, 5-imidazolyl-methyl, 3-guanidino-propyl, phenyl, benzyl, 4-hydroxy-benzyl, 3-indolyl-methyl, (4-hydroxy-3,5-diiodophenoxy)-3,5-diiodobenzyl, thiomethyl, methylthioethyl or a group of formula

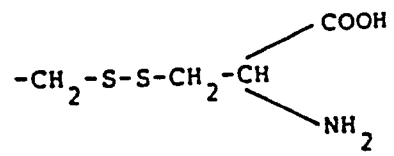
;

when $R_1$ is different from hydrogen, the carbon atoms marked with an asterisk may be in L, D or DL form;

B is an heteroaryl residue selected from the group comprising 3-pyridyl, 5-bromo-3-pyridyl, 5-fluoro-3-pyridyl, pyrazinyl, 5-methyl-pyrazinyl and N-oxides thereof, 5-methyl-pyrazol-3-yl and 5-methyl-isoxazol-3-yl, and pharmaceutically acceptable salts thereof.

2. Compounds of claim 1, wherein $R_1$ is hydrogen.

3. Compounds of claim 1, wherein $R_1$ is methyl, benzyl, methylthioethyl, isopropyl, isobutyl, 2-butyl, phenyl and the carbon atoms marked with an asterisk are in L-form.

4. Compounds of claim 1, wherein B is selected from the group consisting of 3-pyridyl, 5-bromo-3-pyridyl, pyrazinyl or the corresponding N-oxides.

5. 1,10-Bis[2-(nicotinoylglycyl)ethylthio]decane.

6. 1,10-Bis[2-(5'-bromo-nicotinoylglycyl)ethylthio]decane.

7. 1,10-Bis[2-(nicotinoylalanyl)ethylthio]decane.

8. A process for the preparation of compounds of formula I, which consists in reacting 1,10-bis[2-(hydroxyethyl)thio]decane (II)

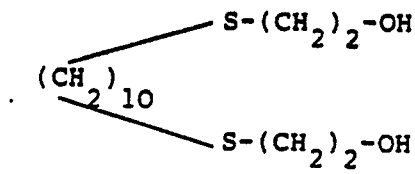
(II)

with a compound of formula (III)

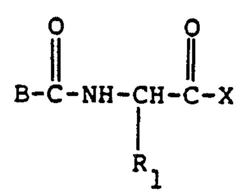
(III)

wherein B and $R_1$ have the above mentioned meanings and X is OH or N,N-bis(2-oxo-3-oxazolidinyl)phosphorodiamidyl.

9. A process according to claim 8, wherein X is OH, which is carried out in the presence of carbodiimides and catalytic amounts of 4-dimethylaminopyridine, in aprotic solvents.

10. A process for the preparation of compounds of formula I, which consists in reacting compounds of formula IV

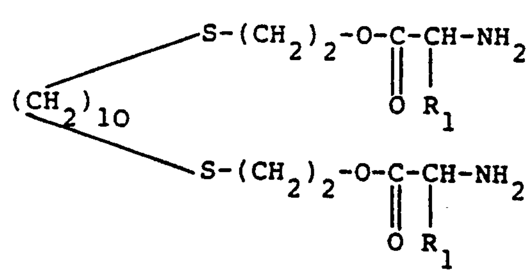
(IV)

wherein $R_1$ has the above mentioned meanings, with a compound of formula V

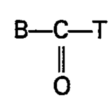
(V)

wherein B has the above mentioned meanings and T is OH, Cl, $N_3$, N,N-bis(2-oxo-3-oxazolidinyl)phosphorodiamidyl

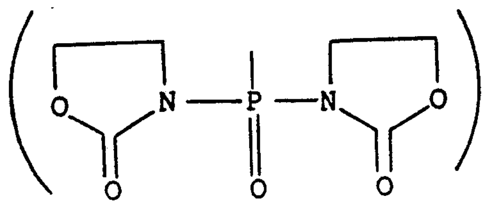, 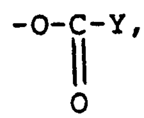

Y being $C_1$—$C_5$ alkoxy, benzyloxy, p-nitrophenoxy, hydroxysuccinimido, hydroxybenzotriazole.

11. A process according to claim 10, wherein T is Cl, which is carried out in toluene, in the presence of tertiary organic bases.

12. Pharmaceutical compositions having hypolipemic, profibrinolytic, antilipolytic and antithrombotic activites, containing as active ingredient one or more compounds of claims 1—7 or salts thereof, with one or more pharmaceutically acceptable excipients.

**Claims for the Contracting State: AT**

1. A process for the preparation of compounds of formula (I)

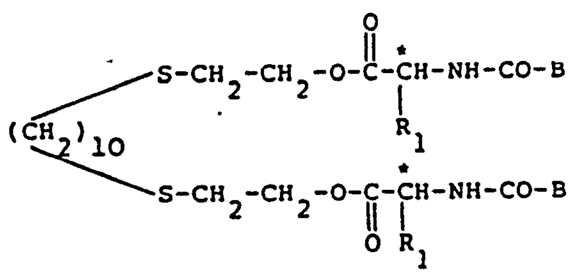

(I)

wherein $R_1$ is hydrogen, methyl, ethyl, isopropyl, isobutyl, 2-butyl, hydroxymethyl, 1-hydroxy-ethyl, carboxymethyl, aminocarbonylmethyl, 2-carboxy-ethyl, 2-aminocarbonylethyl, 4-amino-butyl, 4-amino-3-hydroxy-butyl, 5-imidazolyl-methyl, 3-guanidino-propyl, phenyl, benzyl, 4-hydroxy-benzyl, 3-indolyl-methyl, (4-hydroxy-3,5-diiodophenoxy)-3,5-diiodobenzyl, thiomethyl, methylthioethyl or a group of formula

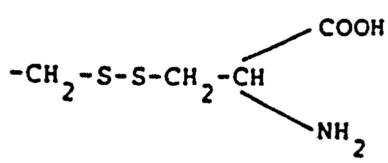 ;

when $R_1$ is different from hydrogen, the carbon atoms marked with an asterisk may be in L, D or DL form;

B is an heteroaryl residue selected from the group comprising 3-pyridyl, 5-bromo-3-pyridyl, 5-fluoro-3-pyridyl, pyrazinyl, 5-methyl-pyrazinyl and N-oxides thereof, 5-methyl-pyrazol-3-yl and 5-methyl-isoxazol-3-yl, and pharmaceutically acceptable salts thereof, which consists in reacting 1,10-bis[2-(hydroxyethyl)thio]decane (II)

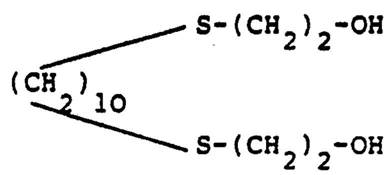

(II)

with a compound of formula (III)

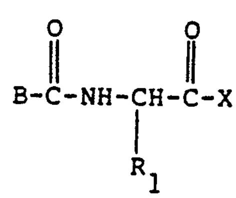

(III)

wherein B and $R_1$ have the above mentioned meanings and X is OH or N,N-bis(2-oxo-3-oxazolidinyl)phosphorodiamidyl.

2. A process according to claim 1, wherein X is OH, which is carried out in the presence of carbodiimides and catalytic amounts of 4-dimethylaminopyridine, in aprotic solvents.

3. A process for the preparation of compounds of formula I, which consists in reacting compounds of formula IV

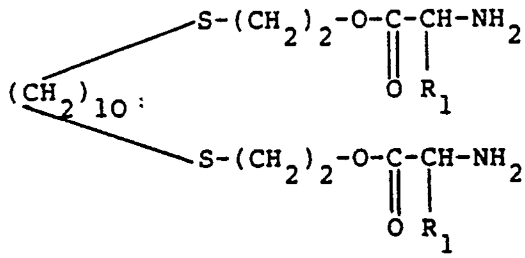

(IV)

wherein $R_1$ has the above mentioned meanings, with a compound of formula V

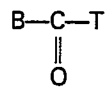

(V)

wherein B has the above mentioned meanings and T is OH, Cl, $N_3$, N,N-bis(2-oxo-3-oxazolidinyl)phosphorodiamidyl

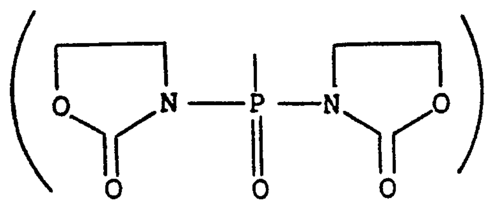

,

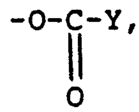

Y being $C_1$—$C_5$ alkoxy, benzyloxy, p-nitrophenoxy, hydroxysuccinimido, hydroxybenzotriazole.

4. A process according to claim 3, wherein T is Cl, which is carried out in toluene, in the presence of tertiary organic bases.

**Patentansprüche für die Vertragsstaaten: BE CH DE FR GB IT LI LU NL SE**

1. Verbindungen der allgemeinen Formel (I)

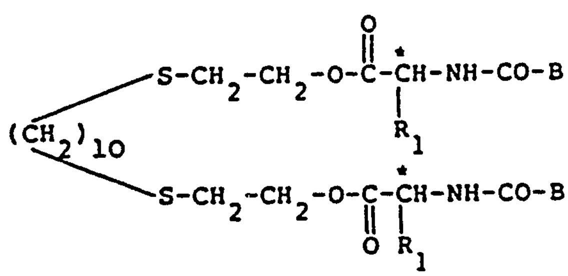

(I)

in denen $R_1$ ein Wasserstoffatom, eine Methyl-, Äthyl-, Isopropyl-, Isobutyl-, 2-Butyl-, Hydroxymethyl-, 1-Hydroxy-äthyl-, Carboxymethyl-, Aminocarbonylmethyl-, 2-Carboxy-äthyl-, 2-Aminocarbonyläthyl-, 4-Amino-butyl-, 4-Amino-3-hydroxy-butyl-, 5-Imidazolyl-methyl-, 3-Guanidino-propyl-, Phenyl-, Benzyl-, 4-Hydroxy-benzyl-, 3-Indolyl-methyl-, (4-Hydroxy-3,5-dijodophenoxy)-3,5-dijodobenzyl-, Thiomethyl-, Methylthioäthylgruppe oder eine Gruppe der Formel

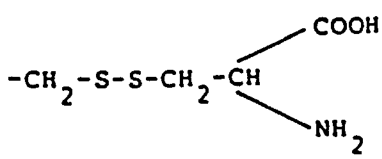

;

darstellt;

wobei, wenn $R_1$ eine andere Bedeutung als ein Wasserstoffatom hat, die mit einem Sternchen (*) markierten Kohlenstoffatome in L-, D- oder DL-Form, vorliegen;

B einen Heteroarylrest bedeutet, der aus der Gruppe gewählt wird, die die folgenden Reste umfaßt: 3-Pyridyl-, 5-Bromo-3-pyridyl-, 5-Fluor-3-pyridyl-, Pyrazinyl-, 5-Methyl-pyrazinylgruppe und deren N-Oxide, die 5-Methyl-pyrazol-3-yl- und 5-Methyl-isoxazol-3-ylgruppe; sowie ihre pharmazeutisch verträglichen Salze.

2. Verbindungen nach Anspruch 1, in denen $R_1$ ein Wasserstoffatom bedeutet.

3. Verbindungen nach Anspruch 1, in denen $R_1$ eine Methyl-, Benzyl-, Methylthioäthyl-, Isopropyl-, Isobutyl-, 2-Butyl- und Phenylgruppe darstellt und die mit einem Sternchen markierten Kohlenstoffatome in L-form vorliegen.

4. Verbindungen nach Anspruch 1, in denen B aus der Gruppe 3-Pyridyl, 5-Brom-3-pyridyl, und Pyrazinyl oder den entsprechenden N-Oxiden gewählt wird.

5. 1,10-Bis-[2-(Nicotinoylglycyl)-äthylthio]-decan.

6. 1,10-Bis-[2-(5'-brom-nicotinoylglycyl)-äthylthio]-decan.

7. 1,10-Bis-[2-Nicotinoylalanyl)-äthylthio]-decan.

8. Verfahren zur Herstellung von Verbindungen der allgemeinen Formel I, bestehend aus der Umsetzung von 1,10-Bis-[2-(hydroxyäthyl)-thio]-decan (II)

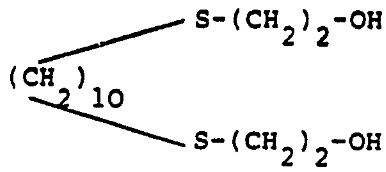

(II)

mit einer Verbindung der allgemeinen Formel (III)

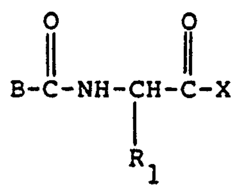

(III)

wobei B und $R_1$ die vorstehend genannten Bedeutungen aufweisen und X eine Hydroxy- oder eine N,N-Bis-(2-oxo-3-oxazolidinyl)-phosphordiamidylgruppe bedeutet.

9. Verfahren nach Anspruch 8, in dem X eine Hydroxygruppe darstellt, das in Gegenwart von Carbodiimiden und katalytischen Mengen von 4-Dimethylaminopyridin in aprotischen Lösungsmitteln durchgeführt wird.

10. Verfahren zur Herstellung von Verbindungen der allgemeinen Formel I, bestehend aus der Umsetzung von Verbindungen der allgemeinen Formel IV

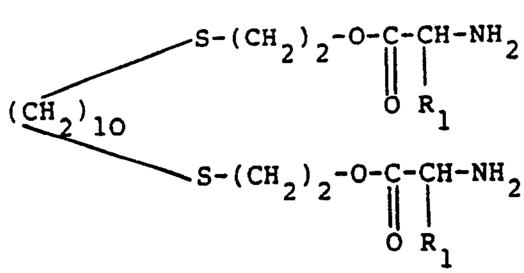

(IV)

in der $R_1$ die vorstehend genannten Bedeutungen aufweist, mit einer Verbindung der allgemeinen Formel V

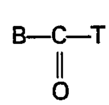

(V)

wobei B die vorstehend genannten Bedeutungen aufweist und T OH, Cl, $N_3$ oder eine N,N-Bis-(2-oxo-3-oxazolidinyl)-phosphordiamidylgruppe ist oder einen Rest der Formel

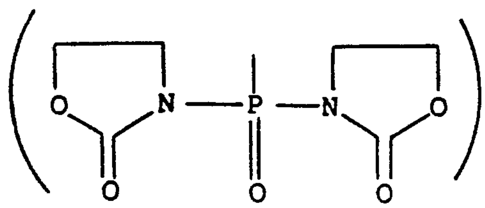, oder 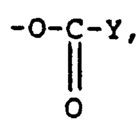,

darstellt,

in dem Y einen $C_1$—$C_5$-Alkoxyrest, eine Benzyloxy-, p-Nitrophenoxy-, Hydroxysuccinimido- oder Hydroxybenzotriazolgruppe bedeutet.

11. Verfahren nach Anspruch 10, das in Toluol in Gegenwart von tertiären organischen Basen durchgeführt wird und in dem T ein Chloratom darstellt.

12. Arzneimittel mit hypolipämischen, profibrinolytischen, antilipolytischen und antithrombotischen Wirkungen, die als Wirkstoff eine oder mehrere Verbindungen nach Anspruch 1 bis 7 oder deren Salze enthalten, sowie einen oder mehrere pharmazeutisch verträgliche Exzipientien.

**Patentansprüche für den Vertragsstaat: AT**

1. Verfahren zur Herstellung von Verbindungen der allgemeinen Formel (I)

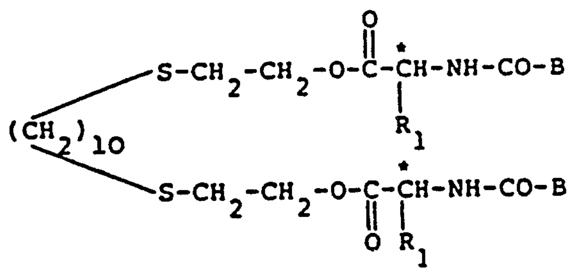

(I)

in denen $R_1$ ein Wasserstoffatom, eine Methyl-, Äthyl-, Isopropyl-, Isobutyl-, 2-Butyl-, Hydroxymethyl-, 1-Hydroxy-äthyl-, Carboxymethyl-, Aminocarbonylmethyl-, 2-Carboxy-äthyl-, 2-Aminocarbonyläthyl-, 4-Amino-butyl-, 4-Amino-3-hydroxy-butyl-, 5-Imidazolyl-methyl-, 3-Guanidino-propyl-, Phenyl-, Benzyl-, 4-Hydroxy-benzyl-, 3-Indolyl-methyl-, (4-Hydroxy-3,5-dijodophenoxy)-3,5-dijodobenzyl-, Thiomethyl-, Methylthioäthylgruppe oder eine Gruppe der Formel

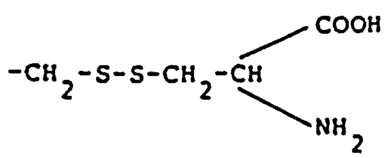 ;

darstellt;

wobei, wenn $R_1$ eine andere Bedeutung als ein Wasserstoffatom hat, die mit einem Sternchen (*) markierten Kohlenstoffatome in L-, D- oder DL-Form, vorliegen;

B einen Heteroarylrest bedeutet, der aus der Gruppe gewählt wird, die die folgenden Reste umfaßt: 3-Pyridyl-, 5-Bromo-3-pyridyl-, 5-Fluor-3-pyridyl-, Pyrazinyl-, 5-Methyl-pyrazinylgruppe und deren N-Oxide, die 5-Methyl-pyrazol-3-yl- und 5-Methyl-isoxazol-3-ylgruppe; sowie ihre pharmazeutisch verträglichen Salze,

bestehend aus der Umsetzung von 1,10-Bis-[2-(hydroxyäthyl)-thio]-decan (II)

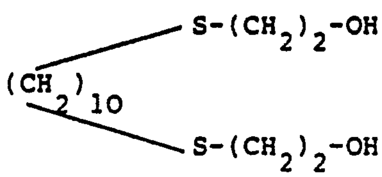

(II)

mit einer Verbindung der allgemeinen Formel (III)

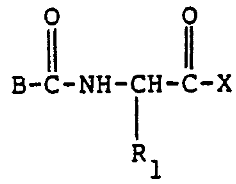

(III)

wobei B und $R_1$ die vorstehend genannten Bedeutungen aufweisen und X eine Hydroxy- oder eine N,N-Bis-(2-oxo-3-oxazolidinyl)-phosphorodiamidylgruppe bedeutet.

2. Verfahren nach Anspruch 1, in dem X eine Hydroxygruppe darstellt, das in Gegenwart von Carbodiimiden und katalytischen Mengen von 4-Dimethylaminopyridin in aprotischen Lösungsmitteln durchgeführt wird.

3. Verfahren zur Herstellung von Verbindungen der allgemeinen Formel I, bestehend aus der Umsetzung von Verbindungen der allgemeinen Formel IV

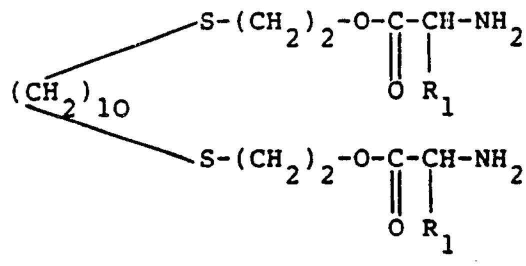

(IV)

in der $R_1$ die vorstehend genannten Bedeutungen aufweist, mit einer Verbindung der allgemeinen Formel V

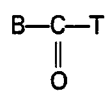

(V)

wobei B die vorstehend genannten Bedeutungen aufweist und T OH, Cl, $N_3$ oder eine N,N-Bis-(2-oxo-3-oxazolidinyl)-phosphordiamidylgruppe ist oder einen Rest der Formel

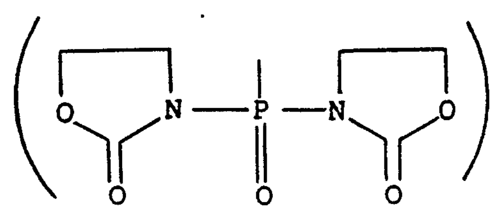

, oder

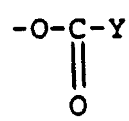

,

darstellt,

in dem Y einen $C_1$—$C_5$-Alkoxyrest, eine Benzyloxy-, p-Nitrophenoxy-, Hydroxysuccinimido- oder Hydroxybenzotriazolgruppe bedeutet.

4. Verfahren nach Anspruch 3, das in Toluol in Gegenwart von tertiären organischen Basen durchgeführt wird und in dem T ein Chloratom darstellt.

**Revendications pour les Etats contractants: BE CH DE FR GB IT LI LU NL SE**

1. Composés de formule (I):

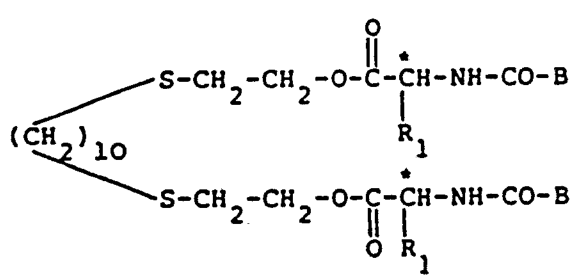

(I)

dans laquelle:

$R_1$ représente hydrogène, méthyle, éthyle, isopropyle, isobutyle, butyle-2, hydroxyméthyle, hydroxy-1

éthyle, carboxyméthyle, aminocarbonylméthyle, carboxy-2 éthyle, aminocarbonyl-2 éthyle, amino-4 butyle, amino-4 hydroxy-3 butyle, imidazolyl-5 méthyle, guanidino-3 propyle, phényle, benzyle, hydroxy-4 benzyle, indolyl-3 méthyle, (hydroxy-4 diiodo-3,5 phénoxy)-diiodo-3,5 benzyle, thiométhyle, méthylthioéthyle ou un groupe de formule:

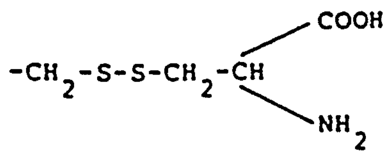 ;

lorsque $R_1$ ne représente pas hydrogène, les atomes de carbone marqués d'un astérisque peuvent se présenter sous la forme L, D ou DL;

B représente un reste hétéroaryle choisi parmi pyridyle-3, bromo-5 pyridyle-3, fluoro-5 pyridyle-3, pyrazinyle, méthyl-5 pyrazinyle et leurs N-oxydes, méthyl-5 pyrazolyle-3 et méthyl-5 isoxazolyle-3,
et leurs sels pharmaceutiquement acceptables.

2. Composés selon la revendication 1, dans lesquels $R_1$ représente hydrogène.

3. Composés selon la revendication 1, dans lesquels $R_1$ représente méthyle, benzyle, méthylthioéthyle, isopropyle, isobutyle, butyle-2, phényle et les atomes de carbone marqués d'un astérisque se présentant sous la forme L.

4. Composés selon la revendication 1, dans lesquels B est choisi parmi pyridyle-3, bromo-5 pyridyle-3, pyrazinyle ou les N-oxydes correspondants.

5. Bis[(nicotinoylglycyl)-2 éthylthio]-1,10 décane.

6. Bis[(bromo-5' nicotinoylglycyl)-2 éthylthio]-1,10 décane.

7. Bis[(nicotinoylalanyl)-2 éthylthio]-1,10 décane.

8. Procédé de préparation de composés de formule (I), qui consiste à faire réagir le bis[(hydroxy)-2 éthylthio]-1,10 décane (II):

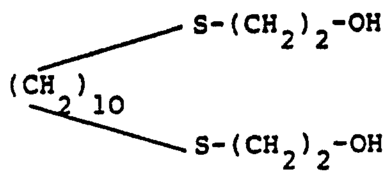 (II)

avec un composé de formule (III):

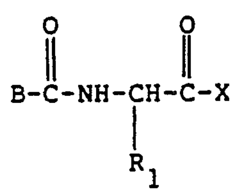 (III)

dans laquelle:

B et $R_1$ ont les significations mentionnées ci-dessus; et

X représente OH ou N,N-bis(oxo-2 oxazolidinyl-3)-phorphorodiamidyle.

9. Procédé selon la revendication 8, dans lequel X représente OH, qui est conduit, en présence de carbodiimides et de quantités catalytiques de diméthylamino-4 pyridine, dans des solvants aprotiques.

10. Procédé de préparation de composés de formule (I), qui consiste à faire réagir des composés de formule (IV):

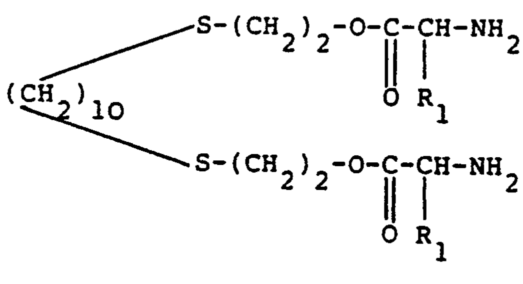 (IV)

dans laquelle $R_1$ a les significations mentionnées ci-dessus, avec un composé de formule (V):

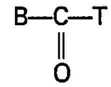 (V)

dans laquelle:

B a les significations mentionnées ci-dessus; et

T représente OH, Cl, $N_3$, N,N-bis(oxo-2 oxazolidinyl-3)phosphorodiamidyle,

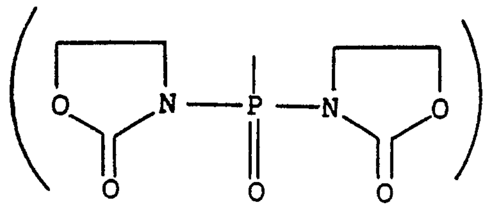,

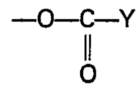

Y représentant alcoxy en $C_1$—$C_5$, benzyloxy, p-nitrophénoxy, hydroxysuccinimido, hydroxybenzotriazole.

11. Procédé selon la revendication 10, dans lequel T représente Cl, qui est conduit dans du toluène, en présence de bases organiques tertiaires.

12. Compositions pharmaceutiques présentant des activité hypolipidémiques, profibrinolytiques, antilipolytiques et antithrombotiques, contenant, en tant qu'ingrédient actif, un ou plusieurs composés tels que définis aux revendications 1—7, ou leurs sels, avec un ou plusieurs excipients pharmaceutiquement acceptables.

**Revendications pour l'Etat contractant: AT**

1. Procédé de préparation de composés de formule (I):

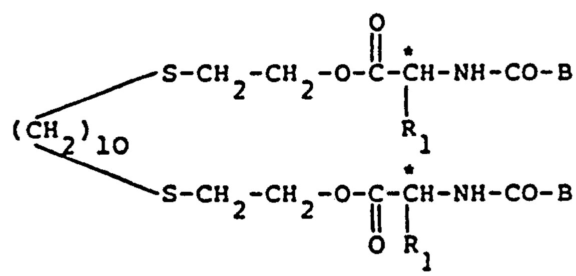 (I)

dans laquelle:

$R_1$ représente hydrogène, méthyle, éthyle, isopropyle, isobutyle, butyle-2, hydroxyméthyle, hydroxy-1 éthyle, carboxyméthyle, aminocarbonylméthyle, carboxy-2 éthyle, aminocarbonyl-2 éthyle, amino-4 butyle, amino-4 hydroxy-3 butyle, imidazolyl-5 méthyle, guanidino-3 propyle, phényle, benzyle, hydroxy-4 benzyle, indolyl-3 méthyle, (hydroxy-4 diiodo-3,5 phénoxy)-diiodo-3,5 benzyle, thiométhyle, méthylthioéthyle ou un groupe de formule:

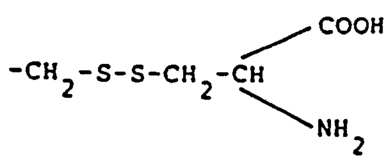 ;

lorsque $R_1$ ne représente pas hydrogène, les atomes de carbone marqués d'un astérisque peuvent se présenter sous la forme L, D ou DL;

B représente un reste hétéroaryle choisi parmi pyridyle-3, bromo-5 pyridyle-3, fluoro-5 pyridyle-3, pyrazinyle, méthyl-5 pyrazinyle et leurs N-oxydes, méthyl-5 pyrazolyle-3 et méthyl-5 isoxazolyle-3, et leurs sels pharmaceutiquement acceptables, qui consiste à faire réagir le bis[(hydroxy)-2 éthylthio]-1,10 décane (II):

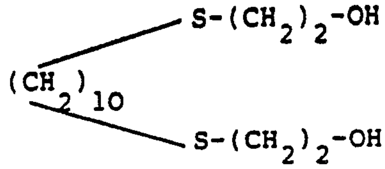

(II)

avec un composé de formule (III)

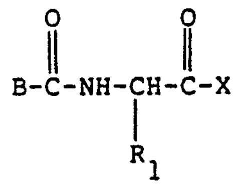

(III)

dans laquelle:

B et $R_1$ ont les significations mentionnées ci-dessus; et

X représente OH ou N,N-bis(oxo-2 oxazolidinyl-3)-phosphorodiamidyle.

2. Procédé selon la revendication 1, dans lequel X représente OH, qui est conduit, en présence de carbodiimides et de quantités catalytiques de diméthylamino-4 pyridine, dans des solvants aprotiques.

3. Procédé de préparation de composés de formule (I), qui consiste à faire réagir des composés de formule (IV):

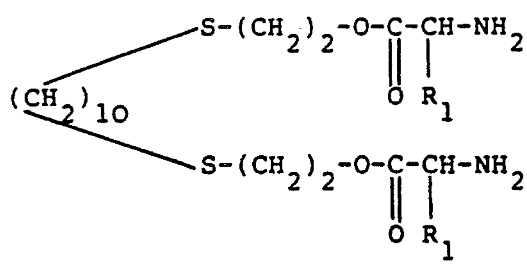

(IV)

dans laquelle $R_1$ a les significations mentionnées ci-dessus, avec un composé de formule (V):

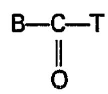

(V)

dans laquelle:

B a les significations mentionnées ci-dessus; et

T représente OH, Cl, $N_3$, N,N-bis(oxo-2 oxazolidinyl-3)phosphorodiamidyle,

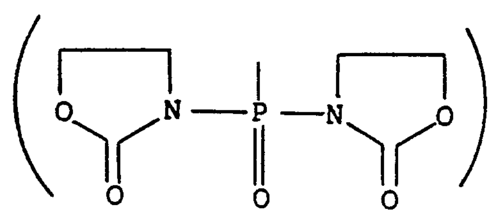

,

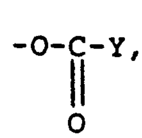

Y représentant alcoxy en $C_1$—$C_5$, benzyloxy, p-nitrophénoxy, hydroxysuccinimido, hydroxybenzotriazole.

4. Procédé selon la revendication 3, dans lequel T représente Cl, qui est conduit dans du toluène, en présence de bases organiques tertiaires.